# EUROPEAN PATENT APPLICATION

(11) **EP 1 834 651 A1**
(43) Date of publication of application: **19.09.2007**
(21) Application number: 06005245.3
(22) Date of filing: 15.03.2006
(51) Int. Cl.: A61K 39/39, A61K 35/76, A61K 39/00

(54) **Compositions and methods for veterinary vaccination**

(71) Applicant: Universiteit Gent, 9000 Gent (BE)
(72) Inventor: Remon, Jean Paul, B-9090 Melle (BE); Vervaet, Chris, B-8870 Izegem (BE); Corbanie, Evy, B-8790 Waregem (BE)
(74) Representative: Bird, William Edward

(57) **Abstract**

This invention relates to powder compositions for veterinary vaccination being especially useful against, but not limited to, avian viral infections, and to a method for producing them. These compositions comprise an effective amount of a veterinary vaccine and a supporting amount of one or more carriers for said veterinary vaccine, said powder being in the form of particles with an average particle size from 2 to 30 µm, and a narrow polydispersity which is retained after nebulization. The compositions are useful for poultry vaccination, especially against Newcastle disease and bird flu.

## Description

### Field of the invention

The present invention relates to compositions for veterinary vaccination useful for inducing protective responses in poultry against avian diseases such as, but not limited to, Newcastle disease and avian influenza. The present invention also relates to methods for producing and using these compositions.

### Background of the invention

Avian infectious diseases are well known for their negative economic impact on poultry production and, possibly, on the health of other animals (in particular mammals) and human beings which may be directly or indirectly in contact with infected birds.

For example specific avian pathogens such as, but not limited to, Newcastle disease virus (hereinafter abbreviated as NDV), infectious bronchitis virus (IBV), infectious bursal disease virus (IBDV), turkey rhinotracheitis virus (TRTV), infectious laryngotracheitis virus (ILTV), egg drop syndrome (EDS) virus, avian encephalomyelitis virus, reticuloendotheleisis virus, avian pox viruses, avian adenoviruses, infectious coryza, fowl typhoid, fowl cholera, avian influenza, Mycoplasma gallisepticum, Escherichia coli, Salmonella spp, etc. may spread from infected poultry.

More specifically, Newcastle disease is a viral infection of poultry with a wide geographical distribution which may cause great economical losses to the poultry industry. NDV is the etiologic agent of said disease and represents the prototype virus of the genus Paramyxovirus. There are nine serotypes of avian paramyxoviruses designated from APMV-I to APMV-9. Newcastle disease is complicated in that different NDV strains may induce very significant variation in the severity of the disease. Infection may take place by either inhalation or ingestion and the infectious form of the virus is then spread from one bird to another.

Avian influenza, or " bird flu ", is a contagious disease of animals caused by Influenza A viruses that normally infect only birds and, less commonly, pigs. Avian influenza viruses tend to be species-specific, but can cross the species barrier to infect mammals and human beings. Influenza A virus have 16 H subtypes. Only viruses of the H5 and H7 subtypes are known to cause the highly pathogenic form of the disease. The H5N1 virus, responsible for the current worldwide epidemic of bird flu has proved to be especially tenacious, and is presently of particular concern for human health.

Spray and aerosol vaccination are commonly used mass vaccination techniques for the protection of poultry against respiratory viral infections. Birds are usually vaccinated a number of times during their lives following the specifications of the manufacturer or tailor made programs in order to optimally prevent disease outbreaks. A so-called primary vaccination (priming) is usually performed in chickens aged below about two to three weeks. This primary vaccination is usually followed by one or more so-called secondary vaccinations (boosters).

Approximately 48 hours after vaccination, vaccination reactions may appear with some respiratory viruses, particularly with NDV. Affected chickens show respiratory symptoms, anorexia, depression, growth retardation and increased mortality. The vaccination reaction may be further complicated by secondary colibacilosis (Escherichia coli infection). In order to prevent this, antibiotic treatments are given to the birds 36 hours after vaccination, which often prove to be efficient. Nevertheless, overuse or misuse of antibiotics may induce bacterial resistance, which may hamper adequate treatment whenever multi-resistant strains aggravate vaccination reactions.

Live viruses of low virulence (lentogenic) or moderate virulence (mesogenic) are commonly used for the vaccination of poultry against Newcastle disease depending on the disease situation. Mesogenic ND vaccine viruses are however suitable only for booster vaccination. However, also in the lentogenic group there is a considerable range in virulence. Commonly used live vaccines include lentogenic strains such as V4, Hitchner B1, F and La Sota, and mesogenic strains such as strain H, Mukteswar, Koinarov and Roakin.

The main advantage of live NDV vaccines is that these can be administered by inexpensive mass application techniques, such as spray and drinking water application. However, as mentioned previously, live vaccines may cause severe vaccination reactions, in particular in the lower respiratory tract after aerosol vaccination. In order to minimize this drawback, it is important to use extremely mild virus, especially as agent for primary vaccination. Nevertheless, even the commonly used mild live NDV strains Hitchner B1 and La Sota can still cause moderate respiratory vaccination reactions. Therefore, there is an urgent need in the art for improving the NDV vaccination as to avoid vaccination reactions in inoculated animals, yet producing a solid immunity against the disease.

For aerosol particles to be efficient it is known that inhaled particles should be deposited in specific areas of the respiratory tract of birds taking into account parameters such as, but not limited to, the species of bird, its age, the size of the inhaled particles, and the susceptibility to post-vaccination reactions.

The presently commercially available nebulizers used for the production of coarse sprays generate a significant proportion of particles that will deposit in the deeper airways, i.e. the particle size distributions around the optimal size is too broad. While this effect is desired for booster vaccinations, it may result in adverse vaccination reactions in younger birds.

Newly produced airborne droplets will evaporate to about 10% of their original size. When generated with the currently available spraying techniques, this phenomenon will hamper adequate vaccination results since:
- during primary vaccination, a significant percentage of the vaccine particles will deposit in the deeper airways of birds whereas it should not, thus explaining the high incidence of respiratory post-vaccination reactions observed;
- due to a broad and uncontrolled particle size distribution, only a low percentage of particles evaporated from droplets is inhaled into the lower airways during second vaccination.

Next to an inappropriate droplet size distribution, the loss of vaccine virus due to inactivation during production, reconstitution and nebulization is a second major reason for the low efficiency of known spray and aerosol vaccinations. Firstly, the vaccine virus, which is generally bred in a liquid environment, e.g. the allantoic cavity of specific pathogen-free (SPF) eggs is freeze dried for storage. Although viruses are less susceptible to environmental inactivating factors in dried form and although stabilizing excipients may be added before drying, there is still some loss of virus concentration during live vaccine production and storage. Before nebulization, the vaccine cake needs to be reconstituted and the virus concentration can be further reduced when traces of disinfectant are present in the water used for reconstitution and due to the temperature sensitivity of the virus in a liquid environment. This inactivation increases further when the vaccine droplets evaporate after dispersion. The large reduction in vaccine concentration during spray and aerosol vaccination may endanger the induction of a sufficient immune response. Furthermore, current vaccines in liquid suspensions suffer from a significant lack of stability.

The object of the present invention is to overcome these disadvantages.

### Summary of the invention

The herein presented invention enables to overcome the drawbacks of the current vaccination technology in a simple and inexpensive manner by providing efficient solid compositions for veterinary vaccination, which may be used for both primer and booster vaccinations.

Firstly, this invention relates to a powder composition for veterinary vaccination comprising an effective amount of a veterinary vaccine and one or more carriers for said veterinary vaccine, wherein said powder is in the form of particles having an average particle size from about 2 to 30 µm. Secondly, the present invention relates to a method of performing vaccinations in poultry, said method comprising a nebulizing step of the above described powder composition over said poultry. Thirdly, the present invention relates to methods and processes for producing the above described powder composition in a convenient and inexpensive manner while using manufacturing equipment standard in poultry vaccinology.

### Brief description of the figure

The single figure shows the particle size distribution (in microns) of an exemplary powder composition for veterinary vaccination according to this invention, in volume percentage both for each fraction (left scale) and for cumulative fractions (right scale).

### Detailed description of the invention

The most relevant features of the present invention are:
- the presence of a supporting amount of one or more carriers for the veterinary vaccine in a powder composition for veterinary vaccination
- the powder is in the form of particles having an average particle size from about 2 to 30 µm, and
- the powder has a relatively narrow particle size polydispersity, e.g. from about 1.1 to 4.0.
While meeting the objectives of the invention, useful compositions may be suitably selected by combining particular sub-ranges of the above stated important parameters, depending upon the type (species) and age of poultry to be vaccinated. For instance, a powder composition with a particle average size from about 10 to 30 µm, preferably from about 15 to 25 µm, or even better around 20 µm, is desired for the first vaccination of chickens aged from one day to about three weeks. On the other hand a powder composition with a particle average size from about 3 to 9 µm, preferably from about 3 to 6 µm, or even better around 5 µm, is preferred for the secondary (booster) vaccination.

A particle size polydispersity as low as possible is an important parameter in the accurate control of the vaccination compositions and methods of this invention, and is a determining parameter in the final success of vaccination. Suitable vaccination efficiency can already be obtained while using a powder composition of the invention wherein the particle size polydispersity is below 3.0, preferably below 2.5, and even more preferably below 2.0. Since polydispersity control is more difficult to achieve when particle size is lower, and since there should be a fair balance between vaccination efficiency and the costs of polydispersity control, in many instances it may be sufficient for the particle size polydispersity to be at least 1.3, or at least 1.5, and even at least 1.7 taking into account the conflicting requirements of manufacturing reproducibility and costs, and vaccination efficiency. Suitable working ranges for the particle size polydispersity of the powder composition of this invention are therefore from about 1.3 to about 3.0, preferably from 1.5 to 2.5.

The proportion of the one or more carriers present in the powder composition is not a limiting feature of this invention, and usually does not influence the manufacturing feasibility of the composition. This proportion may be selected by the skilled person, using knowledge standard in the art, depending on various parameters such as, but not limited to, the technical function(s) and number of carriers, their compatibility, the type and concentration of veterinary vaccine and, optionally, the target average particle size to be achieved (e.g. depending upon the type and age of poultry to be vaccinated). The proportion of said one or more carriers may range from 0.5% to 99.5 % by weight of said composition, preferably from 1% to 99 %, and even possibly from 2% to 98 %.

The kind and nature of said one or more carriers present in the powder composition is not a limiting feature of this invention, and may be selected by the skilled person, using knowledge standard in the art, depending on various parameters such as, but not limited to, the technical function to be performed by each individual carrier, its compatibility with the other carrier(s) (in case of multiple carriers), and cost considerations. Said one or more carriers are preferably of a veterinary acceptable grade and may suitably be selected, but without limitation, from the group consisting of:
- reducing sugars such as, but not limited to, glucose, fructose and alike,
- non-reducing sugars such as, but not limited to, trehalose, sucrose, dextran, chemically modified dextrans and alike,
- polyols such as, but not limited to, mannitol, sorbitol and alike,
- biocompatible polymers such as, but not limited to, polyvinylpyrrolidone, polyvinyl acetate and alike,
- proteins such as, but not limited to, albumin, dried milk serum, casein and alike, and
- lipids such as, but not limited to, DPPC, DSPC and alike.

The use of a non-reducing sugar as a carrier in the powder compositions of this invention is usually preferred over reducing sugars. This preference may also be achieved by the use of a mixture thereof, wherein the proportion of non-reducing sugars in the mixture is predominant.

These one or more preferably veterinary acceptable carriers can be used alone or in combination in order to optimise powder properties. For instance, suitable stabilizing carriers tend to become amorphous upon drying, and/or act as water-replacing materials and/or shield the material by encapsulation, which behaviour may help in preserving the original structure of the virus and in contributing to powder stability over time.

Conventional live and attenuated vaccines as well as recombinant vaccines can suitably be used as veterinary vaccination active agent in the powder compositions for veterinary vaccination (preferably poultry vaccination) of the invention.

Viruses which may suitably be incorporated into a powder composition for veterinary vaccination according to the invention can be obtained by any known conventional methods. Briefly, a susceptible substrate is inoculated with the relevant virus and propagated until the virus replicates to a desired concentration, after which time the virus-containing material is harvested. Any substrate being able to sustain virus replication can suitably be used for performing the present invention, including primary (avian) cell cultures, such as chicken embryo fibroblast cells (CEF) or chicken kidney cells (CK), or mammalian cell lines such as the VERO cell line or baby hamster kidney (BHK) cell line. Preferably, the virus is propagated in specific pathogen free chicken embryos.

More particularly, a suitable substrate on which the relevant virus may be propagated is SPF embryonated eggs. Embryonated eggs can be inoculated with, for example 0.2 ml NDV containing allantoic fluid comprising at least 10² embryo infectious dose ₅₀ (herein abbreviated as EID₅₀) per egg. Preferably, 9-12 day-old embryonated eggs are inoculated with about 10⁵ EID₅₀ and subsequently incubated at 37°C for 2 to 4 days, after which time the ND virus product can be harvested, preferably by collecting the allantoic fluid. The fluid can then be centrifuged for about 10 minutes at 2500*g* , followed by filtering the supernatant through a filter (100 µm).

The present invention also relates to a method of producing the above described powder composition for veterinary vaccination, said method comprising freeze-drying an aqueous formulation comprising a mixture of an effective amount of a veterinary vaccination active agent and a supporting amount of one or more carriers for said veterinary vaccination active agent, and then milling the product of said freeze-drying step such as to provide said powder composition in the form of particles having an average size from about 2 to 30 µm and, optionally, a particle size polydispersity from about 1.1 to 4.0.

In yet another aspect, the present invention relates to a method of producing the above described powder composition for veterinary vaccination, said method comprising spray-drying a liquid phase comprising a mixture of an effective amount of a veterinary vaccine and a supporting amount of one or more carriers for said veterinary vaccine, such as to provide said powder composition in the form of particles having an average size from about 2 µm to about 30 µm and, optionally, a particle size polydispersity from about 1.1 to 4.0. The production method of the invention, preferably spray-drying, may be effected at a temperature ranging from about 30°C to about 170°C. The skilled person understands that, depending upon the type of spray-drying equipment and feed rate used, the spray-drying temperature may be quite different at the inlet and the outlet of the spray-drying device. The spray-drying temperature is not a limiting feature of this aspect of the invention, and conventional temperature ranges are from about 40°C to about 160°C. The spray-drying equipment used in this aspect of the invention is not a critical feature of the invention and may be of any type standard in the art.

The veterinary vaccination powder composition of the present invention is fully inhalable and can therefore be efficiently used for either prophylatic or therapeutic immunisation of poultry, e.g. any type of gallinaceous poultry, and a suitable administration route includes, but is not limited to, nebulization of the powder composition over said poultry.

A further aspect of the invention relates to nebulizing equipment including a powder composition for veterinary vaccination according to the above description. Preferably this equipment includes means for pulsing said powder composition together with a gas. Preferably said gas is air. There is no need for an efficient performance of the invention to use special propellants gases which could be inhaled by the birds and cause problems into the respiratory tract.

Yet a further aspect of the invention relates to a method for performing vaccination in poultry, said method comprising nebulizing a powder composition such as described above over said poultry. When this vaccination step is a primary vaccination (primer) in poultry aged below two to three weeks, said powder composition is preferably in the form of particles having an average size from about 10 to 30 µm, more preferably from about 15 to 25 µm, or even better around 20 µm. When this vaccination is a secondary vaccination (booster) in poultry aged over 2 to 3 weeks, said powder composition is preferably in the form of particles having an average size from about 3 to 9 µm, more preferably around 5 µm.

A " veterinary vaccination composition " as used herein refers to a composition comprising at least one protein, or fragment thereof, which provokes an immune response that protects an animal, in particular a bird, from illness when administered in effective amounts to said animal. For use in a vaccination composition according to the present invention, the veterinary active agent may suitably comprise a live, attenuated or recombinant virus, bacteria, mycoplasma or chlamydophyla.

As an illustration, the following Newcastle disease virus strains may be used:
- lentogenic vaccines such as, but not limited to, Hitchner-B₁, La Sota, V4, NDW, I2 and F, and
- mesogenic vaccines such as, but not limited to, Roakin, Lukteswar and Komarov.

As another illustration, the following avian influenza strain may be used: H5N1 Asia strain.

Immunisation is obtained by inhalation of the composition of the invention by poultry. For instance, chickens are exposed to a cloud of the dry powder composition having the selected average particle size and, optionally, the selected particle size polydispersity. The duration of the immunity may depend upon the vaccination programme chosen. One of the most important considerations affecting vaccination programmes is the level of maternal immunity in young chickens, which may vary considerably from farm to farm, batch to batch, and among individual chickens. For this reason, one or more of several strategies may be employed as follows. Either the birds are not vaccinated until 2-4 weeks of age when most of them will be susceptible, or 1-day-old birds are vaccinated by the application of a coarse spray. Revaccination is then carried out 3-4 weeks later.

Spray-drying is able to deliver dispersible powder particles without the necessity of milling after the spray-drying step. In this process, a liquid virus suspension is rapidly transformed into a dried particulate form of suitable average particle size and suitable particle size distribution by atomisation in a hot drying gaseous medium such as, but not limited to, air. A large air-water interfacial area is created, water evaporates rapidly and drying completes in a matter of a few seconds. During the early stage of drying, where the droplet surface remains at 100% of relative humidity, the surface temperature maintains at the wet-bulb temperature that is significantly lower that the hot air temperature. The virus itself will not be exposed to the high temperatures, thus contributing to the desired stability of the resulting powder formulation. When water is evaporated, the air around the solid virus-containing particle has already cooled down due to moisture uptake. Denaturation of the virus may also be avoided through the use of carriers being commonly used for the stabilization of labile materials, such as reducing sugars, non-reducing sugars, polyols, biocompatible polymers, proteins or lipids, or mixtures thereof in any suitable proportions, as mentioned hereinabove.

If suitably performed with all standard industrial precautions, this spray drying production method is able to reproducibly achieve powder compositions with a water content not above about 4% by weight, preferably not above about 3% by weight, and more preferably not above about 2% by weight at the time of production. If conveniently stored before actual use, the water content of such powder compositions does not significantly increase over time.

Typically, the veterinary vaccine powder composition according to the invention can be administered in a dose of about 10³ to 10⁸ EID₅₀ per animal, preferably in a dose ranging from about 10⁵ to 10⁷ EID₅₀.

The present invention will be described in the following example with respect to a particular working embodiment, but the invention is not limited thereto but only by the claims.

### EXAMPLE - preparation of a dry powder vaccination composition by spray-drying

An aqueous solution containing 4% by weight trehalose and 1% by weight polyvinylpyrollidone as carriers and Newcastle Disease virus (2.10¹² EID₅₀ per gram solids) was spray-dried by making use of a Mobile Minor spray-dryer. A 1 mm two-fluid nozzle was operated at an atomizing air pressure of 1 bar. The inlet temperature was set at 160°C, which resulted, together with a feed rate of 26 ml/minute, in an outlet temperature of 70°C.

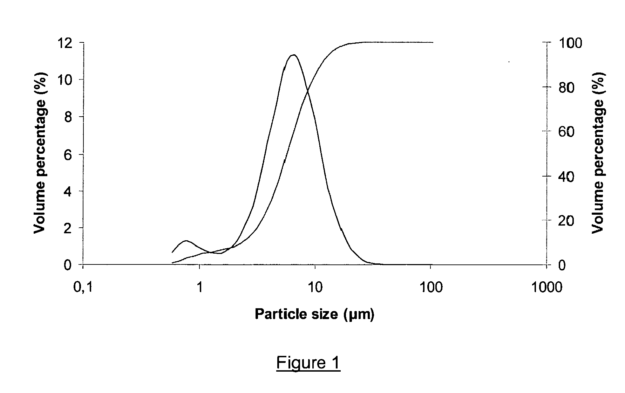

The resulting powder was evaluated:
- for water content and water absorption using Karl Fischer titration,
- for thermodynamic properties with differential scanning calorimetry, and
- for particle size distribution using laser scattering (see Figure 1).

Immediately after production, the water content of the resulting powder was 3.1% by weight, and increased to 5.9% by weight after one week of storage at ambient temperature and relative humidity. The material obtained was completely amorphous with a glass transition temperature of 65°C. The particle size polydispersity of the powder was 1.8, and the median particle size was 6 µm, as shown in Figure 1.

Immediately after drying, an aliquot of the powder was inoculated in SPF eggs and after incubation, the virus content in the allantoic fluids was determined with a haemagglutination test. This showed that more than 90% of the NDV (2.10¹¹ EID₅₀) could be retained per gram of spray dried powder.

*In vivo* evaluation of the dry vaccination powder, performed by exposing chickens to the nebulized dry powder and evaluating the resulting immune response and resistance to challenge with a virulent virus, demonstrates a safe and effective alternative to currently available Newcastle disease vaccines.

## Claims

1. A powder composition for veterinary vaccination comprising an effective amount of a veterinary vaccine and a supporting amount of one or more carriers for said veterinary vaccine, wherein said powder is in the form of particles having a particle average size from about 2 to 30 µm.

2. A powder composition for veterinary vaccination according to claim 1, wherein said powder has a particle size polydispersity from about 1.1 to about 4.0.

3. A powder composition for veterinary vaccination according to claim 1 or claim 2, wherein the proportion of said one or more carriers is from about 0.1 % by weight to about 99.9 % by weight of said composition.

4. A powder composition for veterinary vaccination according to any of claims 1 to 3, wherein said powder is in the form of particles having an average size from about 10 to 30 µm.

5. A powder composition for veterinary vaccination according to any of claims 1 to 4, wherein said one or more carrier(s) is (are) selected from the group consisting of reducing sugars, non-reducing sugars, polyols, biocompatible polymers, proteins and lipids, and mixtures thereof.

6. A method of producing a powder composition for veterinary vaccination according to any of claims 1 to 5, comprising spray-drying a liquid phase comprising a mixture of an effective amount of a veterinary vaccination active agent and a supporting amount of one or more carriers for said veterinary vaccination active agent, such as to provide said powder composition in the form of particles having an average size from about 2 to 30 µm.

7. A method according to claim 6, wherein spray-drying is effected at a temperature ranging from about 30°C to about 170°C.

8. A method according to claim 6 or claim 7, wherein spray-drying is effected in a spray-drying equipment of a type suitable for providing a powder composition having a particle size polydispersity from about 1.1 to about 4.0.

9. A method of performing vaccination in poultry, said method comprising nebulizing a powder vaccination composition according to any of claims 1 to 5 over said poultry.

10. A method according to claim 9, wherein said vaccination is primary vaccination in poultry aged below about two to three weeks, and wherein said powder is in the form of particles having an average particle size from about 10 to 30 µm.

11. A method according to claim 9, wherein said vaccination is secondary vaccination in poultry aged above two to three weeks, and wherein said powder is in the form of particles having an average particle size from about 2 to 5 µm.

12. A system for performing vaccination in poultry comprising:
- a source of a powder vaccination composition according to any of claims 1 to 5, and
- means for nebulizing the powder vaccination composition over said poultry.
